# EUROPÄISCHE PATENTSCHRIFT

(11) **EP 2 057 267 B1**
(45) Veröffentlichungstag und Bekanntmachung des Hinweises auf die Patenterteilung: **02.07.2014**
(21) Anmeldenummer: 07801710.0
(22) Anmeldetag: 16.08.2007
(51) Int. Cl.: C12N 15/10, C12Q 1/68, D21H 21/46

(54) **VERFAHREN ZUM LÖSEN GELADENER NUKLEINSÄURE IN EINER ORGANISCHEN FLÜSSIGKEIT**
METHOD FOR DISSOLVING CHARGED NUCLEIC ACID IN AN ORGANIC LIQUID
PROCÉDÉ POUR DISSOUDRE UN ACIDE NUCLÉIQUE CHARGÉ DANS UN LIQUIDE ORGANIQUE

(30) Priorität: 18.08.2006 DE 102006038791
(43) Veröffentlichungstag der Anmeldung: 13.05.2009
(73) Patentinhaber: Kosak, Hans, 53115 Bonn (DE)
(72) Erfinder: KOSAK, Hans, 53115 Bonn (DE); JOSTEN, André, 90429 Nürnberg (DE)
(74) Vertreter: Ehnis, Tobias
(86) Internationale Anmeldenummer: PCT/EP2007/007257
(87) Internationale Veröffentlichungsnummer: WO 2008/019861

(56) Entgegenhaltungen:
- EP-A- 0 338 591
- EP-A- 1 394 544
- US-A- 5 665 538

## Beschreibung

Die Erfindung betrifft ein Verfahren zum Lösen geladener Nukleinsäure in einer mit Wasser nicht mischbaren organischen Flüssigkeit.

Aus der US 5,665,538 ist es bekannt, DNA zu Erdölmaterial zuzusetzen. Dabei ist die DNA so formuliert, dass sie in dem Erdölmaterial gelöst und daraus durch Waschen mit Wasser im Wesentlichen nicht entfernt werden kann. Das aus der US 5,665,538 bekannte Verfahren dient dazu, den Transport eines Materials, insbesondere eines Erdölprodukts, zu überwachen. Die DNA kann aus dem Erdölprodukt entfernt und durch eine Vervielfältigungsreaktion nachgewiesen werden. Um die DNA in dem Erdölprodukt zu lösen, kann die DNA mit einem hydrophoben Hapten verbunden werden. Eine andere Möglichkeit besteht darin, eine DNA zu verwenden, welche chemisch so verändert ist, dass sie hydrophob ist. Dazu kann die DNA Sulfonukleotide umfassen, die Thiophosphate enthalten, die durch geeignete Agenzien, wie Jodethanol, modifiziert sind. Alternativ kann eine methylierte DNA verwendet werden.

Die genannten Methoden haben den Nachteil, dass die Herstellung der dazu erforderlichen DNA sehr aufwändig ist. Darüber hinaus ist es aufwändig, die DNA danach in die wässrige Phase zu überführen, um sie nachweisen zu können.

Im Falle einer methylierten DNA kann das beispielsweise dadurch erfolgen, dass an deren einem Ende ein Biotinmolekül angeordnet ist, so dass die DNA durch Streptavidin gebunden und dadurch isoliert werden kann. Im Falle einer mit einem hydrophoben Hapten verbundenen DNA, kann die DNA mittels eines für das Hapten spezifischen Antikörpers isoliert werden.

Aus der EP 1 394 544 A1 ist ein Verfahren zum Einmischen von Ribonukleinsäure in wasserunlösliche Medien bekannt. Dabei wird ein wasserunlösliches Medium zunächst in einem Lösemittel gelöst und dann mit der in Wasser gelösten Nukleinsäure gemischt. Beispielsweise kann das wasserunlösliche Medium Polystyrol und das Lösemittel Chloroform sein. Vor dem Mischen des in Chloroform gelösten Polystyrols mit der in Wasser gelösten Nukleinsäure wird der in Wasser gelösten Nukleinsäure eine Zwischenlösung aus 95% Ethanol und Aceton zugesetzt. Die durch das Mischen erhaltene Polystyrollösung enthält Nukleinsäure und kann als fälschungssichere Markierung für Produkte verwendet werden. Nachteilig an diesem Verfahren ist, dass es verhältnismäßig aufwändig ist und dazu Polymere erforderlich sind. Darüber hinaus offenbart die EP 1 394 544 A1 nicht, wie die in dem Polymer enthaltene Nukleinsäure wieder so entfernt werden kann, dass diese nachgewiesen werden kann.

Aufgabe der vorliegenden Erfindung ist es, ein günstiges und wenig aufwändiges Verfahren anzugeben, mit welchem eine geladene Nukleinsäure in einer mit Wasser nicht mischbaren organischen Flüssigkeit gelöst werden kann. Weiterhin soll eine mit Wasser nicht mischbare organische Flüssigkeit, in welcher geladene Nukleinsäure gelöst ist, und eine Verwendung dieser Flüssigkeit angegeben werden. Die Nukleinsäure soll so gelöst werden, dass sie sich problemlos aus der organischen Flüssigkeit wiedergewinnen lässt. Die Verwendung solcher Flüssigkeiten, zum Beispiel in pharmazeutischen Zusammensetzungen sowie die Bereitstellung von Verfahren zur Analyse von Nahrungsmitteln zählen ebenfalls zu den erfindungsgemäßen Aufgaben.

Unter einer geladenen Nukleinsäure ist eine Nukleinsäure zu verstehen, deren Nukleotide durch Phosphodiesterbindungen miteinander verknüpft sind, wobei die an den Phosphodiesterbindungen beteiligten Phosphatreste negativ geladen sind, wie es bei natürlich vorkommender DNA oder RNA der Fall ist. Üblicherweise lösen sich Nukleinsäuren auf Grund ihrer Ladung gut in Wasser, nicht jedoch in mit Wasser nicht mischbaren organischen Flüssigkeiten, wie Kohlenwasserstoffen. Unter einer mit Wasser nicht mischbaren organischen Flüssigkeit wird eine organische Flüssigkeit verstanden, bei der zum Lösen von 1 ml mehr als 1 Liter, vorzugsweise mehr als 10 Liter, Wasser notwendig sind. Im Allgemeinen handelt es sich dabei um eine Flüssigkeit biologischen Ursprungs. Das kann beispielsweise ein Öl oder ein Fett oder Wachs in geschmolzenem Zustand sein, welches pflanzlichen, mineralischen oder tierischen Ursprungs ist. Es kann sich bei der Flüssigkeit auch um einen geschmolzenen Stoff handeln, welcher bei Raumtemperatur üblicherweise als Feststoff vorliegt. Wegen einer möglichen Zerstörung der Nukleinsäure durch Pyrolyse sollte die Temperatur der Flüssigkeit 120 °C, vorzugsweise 100 °C, insbesondere 80 °C nicht übersteigen. Eine Nukleinsäure wird im Sinne der Erfindung als gelöst erachtet, wenn sie sich durch eine 5-minütige Zentrifugation bei 15000 x g nicht abzentrifugieren lässt.

Die Aufgabe wird durch die Merkmale von Anspruch 1 gelöst. Zweckmäßige Ausgestaltungen ergeben sich aus den Merkmalen der Ansprüche 2-21.

In einer weiteren Ausführungsform ist ein Verfahren zum Lösen geladener Nukleinsäuren in einer mit Wasser nicht mischbaren organischen ersten Flüssigkeit mit folgenden Schritten vorgesehen:
a) Bereitstellen einer Lösung der Nukleinsäuren in einer wässrigen zweiten Flüssigkeit,
b) Präzipitieren der Nukleinsäuren durch Zusatz eines mit den Nukleinsäuren in der zweiten Flüssigkeit unlösliche Komplexe bildenden Komplexbildners zu der zweiten Flüssigkeit,
c) Abtrennen der Komplexe von der zweiten Flüssigkeit,
d) Lösen der Komplexe in einer dritten Flüssigkeit, welche aus einer amphiphilen Verbindung besteht oder eine amphiphile Verbindung enthält und
e) Mischen der dritten Flüssigkeit mit der ersten Flüssigkeit.

Die erfindungsgemäßen Verfahren ermöglichen es auf sehr einfache und kostengünstige Weise, eine Lösung geladener Nukleinsäuren in einer mit Wasser nicht mischbaren organischen ersten Flüssigkeit herzustellen. Die Nukleinsäuren können dabei in sehr hoher Konzentration in der ersten Flüssigkeit gelöst werden. Wesentlich ist dafür, dass die Komplexe mit der amphiphilen Verbindung in Kontakt gebracht werden. Ohne die amphiphile Verbindung lassen sich nur verhältnismäßig geringe Mengen der komplexierten Nukleinsäuren in der ersten Flüssigkeit lösen.

Das erfindungsgemäße Verfahren ermöglicht die Markierung der ersten Flüssigkeit mit DNA zur fälschungssicheren Identifizierung. Beispielsweise kann dadurch Erdöl oder ein Erdölprodukt mit einer spezifischen DNA markiert und dessen Transportweg durch Identifizieren der DNA aus einer Probe aus dem Erdöl oder Erdölprodukt überwacht werden. Durch die Möglichkeit, DNA in einer hohen Konzentration in der ersten oder dritten Flüssigkeit zu lösen, kann eine hochkonzentrierte Stammlösung hergestellt werden, welche zur Markierung einer großen Menge von Erdöl oder eines Erdölprodukts, beispielsweise einer Tankerladung, geeignet ist. Weiterhin ermöglicht es das erfindungsgemäße Verfahren, Nukleinsäuren, insbesondere in hohen Konzentrationen, für chemische Reaktionen oder zur Aufbewahrung in mit Wasser nicht mischbaren organischen Flüssigkeiten bereitzustellen. Beispielsweise können dadurch chemische Reaktionen von DNA in Öl ermöglicht werden. Darüber hinaus können Nukleinsäuren in der mit Wasser nicht mischbaren organischen Flüssigkeit ohne Kühlung aufbewahrt und dadurch ohne großen Aufwand über lange Strecken transportiert werden, ohne, insbesondere enzymatisch, abgebaut zu werden. Die Ursache dafür ist vermutlich, dass Nukleinsäure-abbauende Enzyme für deren Aktivität eine wässrige Umgebung benötigen.

Weiterhin ist es dadurch möglich, Nukleinsäuren für pharmazeutische Anwendungen, wie z.B. siRNAs, beispielsweise als Salbe, zu formulieren. Eine solche Formulierung hat den Vorteil sehr langer Haltbarkeit.

Somit betrifft die vorliegende Erfindung auch pharmazeutische Zusammensetzungen, die Nukleinsäuren enthalten, die mit einem Komplexbildner gemäß der Erfindung komplexiert sind und in einer amphiphilen Verbindung vorliegen.

In einer bevorzugten Ausführungsform betrifft die Erfindung eine pharmazeutische Zusammensetzung, die eine mit Wasser nicht mischbare organische Flüssigkeit gemäß der vorliegenden Erfindung umfasst.

In einer weiteren bevorzugten Ausführungsform ist die Nukleinsäure RNA und besonders bevorzugt siRNA.

Die pharmazeutischen Zusammensetzungen werden verwendet, um Nukleinsäuren in einer stabilen Form zu verabreichen. Die erfindungsgemäßen Zusammensetzungen können beispielsweise in Form von Granulaten, Pulvern, Tabletten, Kapseln, Sirup, Suppositorien, Injektionen, Emulsionen, Suspensionen oder Lösungen vorliegen. Die erfindungsgemäßen Zusammensetzungen können für verschiedene Verabreichungsarten formuliert sein, beispielsweise für die topische, orale, bukkale, sublinguale, transmukosale, rektale, subkutane, intrathekale, intravenöse, intramuskuläre, intraperitoneale, nasale, intraokulare oder intraventrikuläre Verabreichung.

In einer bevorzugten Ausführungsform sind die pharmazeutischen Zusammensetzungen für eine topische Applikation ausgestaltet. In einer besonders bevorzugten Ausführungsform liegt die pharmazeutische Zusammensetzung, welche die erfindungsgemäß komplexierte Nukleinsäure umfaßt, in Form einer Salbe vor.

Zur Wiedergewinnung der Nukleinsäure aus der ersten Flüssigkeit kann die Nukleinsäure mittels einem mit Wasser mischbaren Lösungsmittel, z. B. durch Ausschütteln, extrahiert werden, in welchem eine hohe Konzentration an Salzen vorliegt. Beispielsweise kann dazu Natriumazetat-gesättigtes Ethanol oder SDS-gesättigtes Ethanol oder Butanol verwendet werden. Die Nukleinsäure fällt dabei in dem mit Wasser mischbaren Lösungsmittel aus und kann nach Abtrennen des mit Wasser mischbaren Lösungsmittels, z. B. durch Zentrifugation, in Wasser aufgenommen und beispielsweise mittels einer PCR nachgewiesen werden.

Ein weiterer Aspekt der Erfindung betrifft somit Verfahren zur Isolierung von Nukleinsäuren aus einer mit Wasser nicht mischbaren Flüssigkeit.

In einer ersten Ausführungsform umfaßt das Verfahren zur Isolierung von Nukleinsäuren aus einer mit Wasser nicht mischbaren Flüssigkeit Schritte, bei denen man
a) die Nukleinsäuren mit einem mit Wasser mischbaren Lösungsmittel aus der Flüssigkeit extrahiert, indem man die mit Wasser nicht mischbare Flüssigkeit mit dem mit Wasser mischbaren Lösungsmittel in Kontakt bringt, wobei das mit Wasser mischbare Lösungsmittel ein oder mehrere Salz(e) enthält;
b) das mit Wasser mischbare Lösungsmittel von der nicht mit Wasser mischbaren Flüssigkeit trennt; und
c) die Nukleinsäuren aus dem mit Wasser mischbaren Lösungsmittel isoliert.

In einer zweiten Ausführungsform umfaßt das Verfahren zur Isolierung von Nukleinsäuren aus einer mit Wasser nicht mischbaren Flüssigkeit das Mischen dieser Flüssigkeit mit einem Lösungsmittel, das ein oder mehrere Salz(e) enthält und sich in der mit Wasser nicht mischbaren Flüssigkeit löst, wodurch die Nukleinsäuren ausfallen.

Eine weitere Ausführungsform betrifft ein Verfahren zur Isolierung von Nukleinsäuren aus einer mit Wasser nicht mischbaren Flüssigkeit, bei dem man ein oder mehrere Salz(e) zu dieser Flüssigkeit zugibt, wodurch die Nukleinsäuren ausfallen.

Geeignete Salze sind beispielsweise Natriumbromid, Natriumdodecylsulfat (SDS) oder Natriumacetat.

Die Salze werden in einer Menge eingesetzt, die 50%, bevorzugt 60%, mehr bevorzugt 70%, besonders bevorzugt 80% und am meisten bevorzugt 90% der Sättigungslöslichkeit, d.h. der Menge des Salzes entspricht, die unter Standardbedingungen eingesetzt werden muß, um mit dem ausgewählten Lösungsmittel eine gesättigte Lösung zu ergeben. Diese Menge kann vom Fachmann leicht ermittelt werden.

In einer weiteren bevorzugten Ausführungsform werden die Lösungen in Form von gesättigten Lösungen eingesetzt.

Mit Wasser mischbare Lösungsmittel, die in der oben genannten ersten Ausführungsform eingesetzt werden können, sind somit beispielsweise gesättigte Salzlösungen in mit Wasser vollständig mischbaren Alkoholen, z.B. eine gesättigte NaBr-Lösung in Ethanol.

Die Isolierung einer Nukleinsäure aus einer mit Wasser nicht mischbaren Flüssigkeit kann in einer weiteren Ausführungsform der Erfindung eingesetzt werden, um Nahrungsmittel zu analysieren. So ist es möglich, auch geringste Mengen Nukleinsäuren aus Nahrungsmitteln zu isolieren und dadurch die Herkunft der Bestandteile des Nahrungsmittels zu bestimmen. So lassen sich beispielsweise verschiedene Ölsorten aufgrund der Nukleinsäuren identifizieren, die in den Ölen gefunden werden.

Überraschenderweise wurde festgestellt, dass Nukleinsäuren auch aus Nahrungsmittelproben isoliert werden können, von denen angenommen wurde, dass sie keine Nukleinsäuren enthalten. So wurden im Rahmen der vorliegenden Erfindung beispielsweise in gängigen Speiseölen wie Rapsöl, Olivenöl, Sonnenblumenöl, Kürbiskernöl, Sesamöl, Traubenkernöl, Walnussöl, Distelöl und Palmöl Nukleinsäuren gefunden.

Durch Amplifizierung dieser Nukleinsäuren ist es möglich, zu bestimmen, welche Bestandteile in dem Nahrungsmittel vorhanden waren. So lassen sich beispielsweise hochwertige Speiseöle, wie beispielsweise Olivenöle, von minderwertigen Ölen unterscheiden.

Die Erfindung betrifft somit auch Verfahren zur Analyse von Nahrungsmitteln, bei denen man
a) Nukleinsäuren aus einem oder mehreren mit Wasser nicht mischbaren Bestandteil(en) des Nahrungsmittels unter Verwendung eines der oben genannten Verfahren isoliert; und
b) die Nukleinsäuren analysiert.

In einer bevorzugten Ausführungsform erfolgt die Analyse der Nukleinsäuren mittels PCR.

Der Komplexbildner ist vorzugsweise ein kationisches Detergenz oder ein organisches Amin, insbesondere ein quartäres Amin. Bei dem quartären Amin handelt es sich bevorzugt um Cetyltrimethylammoniumbromid (CTAB). CTAB ist für die vorgesehene Komplexierung gut geeignet und sehr preiswert.

Vorzugsweise wird der Komplexbildner der zweiten Flüssigkeit in Schritt b) in gelöster Form zugesetzt. Dadurch wird eine schnellere Präzipitation ermöglicht als beim Zusatz eines ungelösten Komplexbildners.

Bevorzugt sind die Nukleinsäuren synthetisch hergestellte Nukleinsäuren mit bekannter Sequenz. Dadurch lässt sich eine nahezu unbegrenzte Zahl von Codierungen zur fälschungssicheren Markierung bereitstellen. Besonders bevorzugt ist es, wenn dazu die zur Identifizierung verwendeten Nukleinsäuren in einer großen Zahl weiterer in der ersten Flüssigkeit gelöster Nukleinsäuren "verstreckt" werden, so dass durch eine Sequenzierung nicht die spezifisch zur Markierung dienenden Nukleinsäuren ermittelt werden können. Zur Bereitstellung der weiteren Nukleinsäuren kann z.B. Heringsperm-DNA verwendet werden.

Die Nukleinsäuren können jeweils eine Kettenlänge von 5 bis 100 Nukleotiden, bevorzugt 10 bis 80 Nukleotiden, besonders bevorzugt 15 bis 60 Nukleotiden, aufweisen. Bei den Nukleinsäuren kann es sich um DNA, insbesondere Antisinn-DNA, oder RNA, insbesondere siRNA, handeln. Für eine therapeutische Anwendung ist es besonders vorteilhaft, wenn die Nukleinsäuren zumindest einen Teil einer Sequenz eines humanen Gens enthalten.

Die Nukleinsäuren können einzelsträngig oder doppelsträngig ausgebildet sein. Eine doppelsträngig ausgebildete Nukleinsäure kann in Form zweier separater Einzelstränge oder als Haarnadelschleifenstruktur vorliegen.

Das Abtrennen der Komplexe gemäß Schritt c) erfolgt vorzugsweise mittels Zentrifugation oder Filtration. Diese Verfahren stellen eine besonders einfache und effiziente Art der Abtrennung der präzipitierten Komplexe dar.

Bei der amphiphilen Verbindung handelt es sich vorzugsweise um ein organisches Lösungsmittel. Bevorzugt enthält die amphiphile Verbindung mindestens eine Ether-Gruppe und/oder mindestens eine Hydroxyl-Gruppe.

Als Lösungsmittel besonders gut geeignete amphiphile Verbindungen können durch die Formel HO-R1-O-R2 beschrieben werden. Dabei ist R1 und R2 jeweils ein Kohlenwasserstoffrest mit 1 bis 100 Kohlenstoffatomen. Bei der amphiphilen Verbindung kann es sich um Ethylenglycolmonobutylether, Ethylenglycolmonoethylether oder Ethylenglycolmonomethylether handeln.

Bevorzugt werden die Komplexe in der dritten Flüssigkeit in einer solchen Menge gelöst, dass daraus eine Nukleinsäurekonzentration in der dritten Flüssigkeit resultiert, welche größer als 0,1 mg/ml, insbesondere größer als 1 mg/ml, bevorzugt größer als 10 mg/ml, ist. Bei der ersten Flüssigkeit kann es sich um ein pflanzliches oder tierisches Öl oder Fett handeln. Die erste Flüssigkeit kann eine Flüssigkeit sein, welche bei 20°C als Feststoff vorliegt. Es kann sich dabei beispielsweise um ein Wachs handeln.

Die erste Flüssigkeit kann auch ein Treibstoff für einen Verbrennungsmotor sein. Es kann sich bei der ersten Flüssigkeit um ein mineralisches Fett, ein Mineralöl oder ein Mineralöl-Destillat oder Mineralöl-Destillatrückstand, insbesondere Dieselkraftstoff, Leichtöl, Schweröl, Toluol, Benzol oder Benzin, handeln.

Die Erfindung betrifft weiterhin eine mit Wasser nicht mischbare organische Flüssigkeit, welche darin gelöste durch einen Komplexbildner komplexierte geladene Nukleinsäuren und eine amphiphile Verbindung enthält. Die Nukleinsäuren sind dabei synthetisch hergestellte Nukleinsäuren mit bekannter Sequenz. Eine solche Flüssigkeit kann mittels des erfindungsgemäßen Verfahrens hergestellt werden. Die Flüssigkeit eignet sich besonders gut zur Durchführung eines Verfahrens zum Nachweis der Nukleinsäuren zur Identifizierung der Flüssigkeit. Die Identifizierung der Flüssigkeit ist besonders sicher, wenn die zuvor bekannte Sequenz der Nukleinsäure bestätigt wird.

Vorteilhafte Ausgestaltungen der Erfindung ergeben sich aus den folgenden Ausführungsbeispielen.

Soweit nicht anders angegeben, stammen alle Chemikalien von der Fa. Sigma-Aldrich Chemie GmbH, Eschenstrasse 5, 82024 Taufkirchen, Deutschland.

### 1. Präzipitieren von DNA mittels CTAB als Komplexbildner:

10 g Heringsperm-DNA (HS-DNA) wurden in einem Liter TE (10 mmol/l TrisHCl, 1 mmol/l EDTA, pH 8 in entionisiertem Wasser) gelöst. 100 ml einer CTAB-Lösung (100 mmol/l CTAB in entionisiertem Wasser) wurde unter Rühren zugefügt. Der gebildete Niederschlag (CTAB-HS-DNA) wurde abzentrifugiert und bei Raumtemperatur getrocknet.

Weiterhin wurde 1 µMol (entspricht etwa 20 mg) einer synthetisch hergestellten, der Sequenz Nr. 1 gemäß dem anliegenden Sequenzprotokoll entsprechenden, 62 Nukleotide langen, einzelsträngigen DNA (Markierungs-DNA, M-DNA) in 1 ml TE gelöst. 100 µl der CTAB-Lösung wurden unter Rühren zugefügt. Der gebildete Niederschlag (CTAB-M-DNA) wurde abzentrifugiert und bei Raumtemperatur getrocknet.

Zur Herstellung eines sowohl HS-DNA als auch M-DNA enthaltenden Präzipitats wurden 1 g HS-DNA und 1 µMol M-DNA in 100 ml TE gelöst. 10 ml der CTAB-Lösung wurden unter Rühren zugefügt. Der gebildete Niederschlag (CTAB-HS-M-DNA) wurde abzentrifugiert und bei Raumtemperatur getrocknet.

### 2. Löslichkeit der mittels CTAB komplexierten DNA in mit Wasser nicht mischbaren Flüssigkeiten:

Jeweils ca. 1 mg CTAB-HS-DNA wurde in 1,4 ml-Eppendorf-Reaktionsgefäße überführt. In jedes der Eppendorf-Reaktionsgefäße wurde jeweils 1 ml Benzin (Superbenzin, BP), Dieselkraftstoff (BP), Mineralöl, Sonnenblumenöl (Supermarkt), Rapsöl (Supermarkt), Weizenkeimöl (Supermarkt) oder Olivenöl (Supermarkt) hinzugefügt. Anschließend wurden die Reaktionsgefäße kräftig geschüttelt und danach 5 Minuten bei 15000 x g zentrifugiert. In allen Reaktionsgefäßen war ein Niederschlag zu beobachten.

### 3. Löslichkeit der mittels CTAB komplexierten DNA in mit Wasser nicht mischbaren Flüssigkeiten nachdem die komplexierte DNA in Alkohol vorgelöst worden ist:

1 g CTAB-HS-DNA wurde in 50 ml 1-Butanol gelöst. Jeweils 100 µl der Lösung wurde in 1,4 ml-Eppendorf-Reaktionsgefäße überführt. In jedes der Eppendorf-Reaktionsgefäße wurde jeweils 1 ml Benzin, Diesel, Mineralöl, Sonnenblumenöl, Rapsöl, Weizenkeimöl oder Olivenöl hinzugefügt. Anschließend wurden die Reaktionsgefäße kräftig geschüttelt und danach 5 Minuten bei 15000 x g zentrifugiert. In allen Reaktionsgefäßen war ein Niederschlag zu beobachten.

### 4. Löslichkeit der mittels CTAB komplexierten DNA in einer amphiphilen Verbindung:

Jeweils ca. 20 mg CTAB-HS-DNA wurden in 1,4 ml-Eppendorf-Reaktionsgefäße überführt. In jedes der Eppendorf-Reaktionsgefäße wurden jeweils 1000 µl, 500 µl, 200 µl, 100 µl, 50 µl oder 20 µl Ethylenglykolmonobutylether (EGE) hinzugefügt. Anschließend wurden die Reaktionsgefäße kräftig geschüttelt und danach 5 Minuten bei 15000 x g zentrifugiert. In keinem der Reaktionsgefäße war ein Niederschlag zu beobachten. Die CTAB-HS-DNA war vollständig gelöst.

### 5. Löslichkeit der mittels CTAB komplexierten DNA in einer mit Wasser nicht mischbaren Flüssigkeit nachdem die komplexierte DNA in einer amphiphilen Verbindung vorgelöst worden ist:

2 g CTAB-HS-DNA wurden in 50 ml EGE gelöst. Jeweils 100 µl bzw. 500 µl der Lösung wurden in 2 ml-Eppendorf-Reaktionsgefäße überführt. In jedes der Eppendorf-Reaktionsgefäße wurden jeweils 900 µl bzw. 500 µl Benzin, Diesel, Mineralöl, Sonnenblumenöl, Rapsöl, Weizenkeimöl oder Olivenöl hinzugefügt. Anschließend wurden die Reaktionsgefäße kräftig geschüttelt und danach 5 Minuten bei 15000 x g zentrifugiert. In keinem der Reaktionsgefäße war ein Niederschlag zu beobachten. Die CTAB-HS-DNA war vollständig gelöst.

### 6. Extraktion von in mit Wasser nicht mischbaren Flüssigkeiten gelöster DNA:

20 mg CTAB-HS-M-DNA wurden in 1 ml EGE gelöst. Je 100 µl davon wurden in 100 ml Benzin, Diesel, Mineralöl, Sonnenblumenöl oder Rapsöl gelöst. Zur Extraktion der DNA wurde jeweils 1 ml der entstandenen Lösung in ein 1,4 ml-Eppendorf-Reaktionsgefäß überführt. Dazu wurden jeweils 50 µl SDS-gesättigtes Ethanol hinzugefügt, kräftig geschüttelt und 5 Minuten bei 15000 x g zentrifugiert. Der Überstand wurde verworfen. Zu dem entstandenen Pellet wurde jeweils 1 ml Heptan hinzugefügt. Anschließend wurde das Reaktionsgefäß kräftig geschüttelt und erneut 5 Minuten bei 15000 x g zentrifugiert. Der Überstand wurde verworfen und das Pellet in 1 ml 0,1 TE (1 Teil TE, 9 Teile entionisiertes Wasser) aufgenommen.

Mit je 2,5 µl der so entstandenen wässrigen Lösung von HS-DNA und M-DNA wurde eine Polymerasekettenreaktion (PCR) durchgeführt. Dazu wurden die synthetisch hergestellten, den Sequenzen Nr. 2 und 3 gemäß dem anliegenden Sequenzprotokoll entsprechenden M-DNA-spezifischen Primer 1 und 2 verwendet. Die PCR wurde in einem Gesamtvolumen von 25 µl mit einem Kit (peqGOLD PCR-Master-Mix S) der Firma Peqlab (Carl-Thiersch-Str. 2b, 91052 Erlangen, Deutschland) durchgeführt. Die Primerkonzentration betrug jeweils 200 nM pro Primer und es wurden 30 Zyklen mit einer Annealing-Temperatur von 55°C durchgeführt. Die Analyse erfolgte mittel Gelelektrophores auf 10 % (w/v) Polyacrylamidgelen und anschließender Silberfärbung. In allen Proben konnte die M-DNA nachgewiesen werden. Als Kontrolle wurden Extrakte aus Benzin, Diesel, Mineralöl, Sonnenblumenöl und Rapsöl verwendet, denen keine DNA zugesetzt worden war. Bei den Kontrollen konnte keine M-DNA nachgewiesen werden.

### 7. Stabilität von in mit Wasser nicht mischbaren Flüssigkeiten gelöster DNA:

20 mg CTAB-HS-M-DNA wurde in 1 ml EGE gelöst. Je 100 µl davon wurden in 100 ml Dieselkraftstoff, Mineralöl, Sonnenblumenöl oder Rapsöl gelöst. Davon wurden jeweils Proben ä 1 ml abgenommen und bei 4°C, Raumtemperatur, 40°C und 80°C für 24 Stunden inkubiert. Zur Extraktion der DNA wurden zu den Proben jeweils 50 µl SDS-gesättigtes Ethanol zugefügt, kräftig geschüttelt und 5 Minuten bei 15000 x g zentrifugiert. Der Überstand wurde verworfen. Zu dem entstandenen Pellet wurde jeweils 1 ml Heptan zugesetzt, kräftig geschüttelt und 5 Minuten bei 15000 x g zentrifugiert. Der Überstand wurde verworfen und das Pellet in 1 ml 0,1 TE aufgenommen. Mit je 2,5 µl der so entstandenen wässrigen Lösung von HS-DNA und M-DNA wurde eine Polymerasekettenreaktion (PCR) durchgeführt. Dazu wurden die synthetisch hergestellten Primer 1 und 2 verwendet. Die PCR wurde in einem Gesamtvolumen von 25 µl mit dem oben genannten Kit der Firma Peqlab durchgeführt. Die Primerkonzentration betrug jeweils 200 nM pro Primer und es wurden 30 Zyklen mit einer Annealing-Temperatur von 55°C durchgeführt. Die Analyse erfolgte mittel Gelelektrophores auf 10 % (w/v) Polyacrylamidgelen und anschließender Silberfärbung. In allen Proben konnte die M-DNA nachgewiesen werden. Als Kontrolle wurden Extrakte aus Benzin, Diesel, Mineralöl, Sonnenblumenöl und Rapsöl verwendet, denen keine DNA zugesetzt worden war. Bei den Kontrollen konnte keine M-DNA nachgewiesen werden. Das Experiment zeigt, dass in mit Wasser nicht mischbarer Flüssigkeit gelöste DNA auch ohne Kühlung und sogar bei erhöhter Temperatur über einen langen Zeitraum stabil ist.

### 8. Einführen von durch CTAB komplexierter und in EGE vorgelöster DNA in Feststoffe:

10 g Kokosfett (Supermarkt) wurden durch Erhitzen verflüssigt. Nach Abkühlen auf ca. 50 °C wurden 100 µl 2% (w/v) CTAB-HS-M-DNA in EGE zugefügt und gemischt. Das Fett wurde durch Abkühlen auf 4°C verfestigt.

### 9. Extraktion von DNA aus Diesel / Benzin / Raps- / Sonnenblumenöl ohne DNA-Zusatz

Zu je 2 1 ml Diesel-, Benzin-, Rapsöl und Sonnenblumenöl-Proben in 1,4ml Kunststoffreaktionsröhrchen wurden je 100 µl SDS-gesättigtes Ethanol bei Raumtemperatur zugefügt, die Proben 30 Sekunden geschüttelt und danach 1 Std bei 4°C inkubiert. Zur Phasentrennung wurden die Proben bei 4°C und max. Beschleunigung (ca. 16.000 x g) für 15 min zentrifugiert. In allen Proben war ein leichtes Pellet sichtbar. Die flüssige Phase wurde abgezogen und das Pellet mit 1ml n-Heptan versetzt. Die Proben wurden ca. 20-30 Sek. gemischt und anschließend bei 4°C und max. Beschleunigung (ca. 16.000 x g) für 2 min zentrifugiert. Der Überstand wurde abgezogen und verworfen. Die Reaktionsröhrchen wurden zur Trocknung des Pellets auf den Kopf gestellt und ca. 10-20 min stehen gelassen. Das Pellet wurde in 100µl 0.1 x TE mit 0,02 % Tween-20 gelöst. Der DNA-Gehalt in den Proben wurde mittels PicoGreen-Test unter Verwendung einer Eichkurve von Lambda-DNA bestimmt und nach Angaben des Herstellers (PicoGreen^{®} dsDNA Quantitation Kit, Catalog Number - P11496, Invitrogen GmbH, Technologiepark Karlsruhe, Emmy-Noether Strasse 10, 76131 Karlsruhe) durchgeführt. In allen Proben konnte Nukleinsäure nachgewiesen werden. Die Konzentration lag zwischen 1 und 7 ng/ml (siehe Tabelle 1).

**Tabelle 1: DNA-Gehalt in verschiedenen Ölsorten**

| **Probe** | **DNA-Gehalt in ng/ml** |
|---|---|
| Benzin 1 | 2,7 |
| Benzin 2 | 2,4 |
| Diesel 1 | 6,9 |
| Diesel 2 | 6,8 |
| Raps 1 | 2,4 |
| Raps 2 | 3,0 |
| Sonnenbl. 1 | 1,3 |
| Sonnenbl. 2 | 1,6 |

## Patentansprüche

1. Verfahren zum Lösen geladener Nukleinsäuren in einer mit Wasser nicht mischbaren organischen ersten Flüssigkeit mit folgenden Schritten:
a) Bereitstellen einer Lösung der Nukleinsäuren in einer wässrigen zweiten Flüssigkeit,
b) Präzipitieren der Nukleinsäuren durch Zusatz eines mit den Nukleinsäuren in der zweiten Flüssigkeit unlösliche Komplexe bildenden Komplexbildners zu der zweiten Flüssigkeit,
c) Abtrennen der Komplexe von der zweiten Flüssigkeit,
d) Lösen der Komplexe in einer dritten Flüssigkeit, welche aus einer amphiphilen Verbindung besteht oder eine amphiphile Verbindung enthält, und
e) Mischen der dritten Flüssigkeit enthaltend die Nukleinsäuren mit der ersten Flüssigkeit.

2. Verfahren nach Anspruch 1, wobei der Komplexbildner ein kationisches Detergenz oder ein organisches Amin, insbesondere ein quartäres Amin, ist.

3. Verfahren nach Anspruch 2, wobei das quartäre Amin Cetyltrimethylammoniumbromid (CTAB) ist.

4. Verfahren nach einem der Ansprüche 2-3, wobei der Komplexbildner der zweiten Flüssigkeit in Schritt b) in gelöster Form zugesetzt wird.

5. Verfahren nach einem der vorherigen Ansprüche, wobei die Nukleinsäuren synthetisch hergestellte Nukleinsäuren mit bekannter Sequenz sind.

6. Verfahren nach einem der vorherigen Ansprüche, wobei die Nukleinsäuren jeweils eine Kettenlänge von 5 bis 100 Nukleotiden, bevorzugt 10 bis 80 Nukleotiden, besonders bevorzugt 15 bis 60 Nukleotiden, aufweisen.

7. Verfahren nach einem der vorherigen Ansprüche, wobei die Nukleinsäuren aus DNA oder aus RNA bestehen.

8. Verfahren nach einem der vorherigen Ansprüche, wobei die Nukleinsäuren Antisinn-DNAs oder siRNAs sind.

9. Verfahren nach einem der vorherigen Ansprüche, wobei die Nukleinsäuren zumindest einen Teil einer Sequenz eines humanen Gens enthalten.

10. Verfahren nach einem der vorherigen Ansprüche, wobei die Nukleinsäuren einzelsträngig oder doppelsträngig ausgebildet sind.

11. Verfahren nach einem der vorherigen Ansprüche, wobei das Abtrennen der Komplexe gemäß Schritt c) mittels Zentrifugation oder Filtration erfolgt.

12. Verfahren nach einem der vorherigen Ansprüche, wobei die amphiphile Verbindung ein organisches Lösungsmittel ist.

13. Verfahren nach einem der vorherigen Ansprüche, wobei die amphiphile Verbindung mindestens eine Ether-Gruppe enthält.

14. Verfahren nach einem der vorherigen Ansprüche, wobei die amphiphile Verbindung mindestens eine Hydroxyl-Gruppe enthält.

15. Verfahren nach einem der vorherigen Ansprüche, wobei die amphiphile Verbindung durch die Formel HO-R1-O-R2 beschrieben werden kann, wobei R1 und R2 jeweils ein Kohlenwasserstoffrest mit 1 bis 100 Kohlenstoffatomen ist.

16. Verfahren nach einem der vorherigen Ansprüche, wobei die amphiphile Verbindung Ethylenglycolmonobutylether, Ethylenglycolmonoethylether oder Ethylenglycolmonomethylether ist.

17. Verfahren nach einem der vorherigen Ansprüche, wobei die Komplexe in der dritten Flüssigkeit in einer solchen Menge gelöst werden, dass daraus eine Nukleinsäurekonzentration in der dritten Flüssigkeit resultiert, welche größer als 0,1 mg/ml, insbesondere größer als 1 mg/ml, bevorzugt größer als 10 mg/ml, ist.

18. Verfahren nach einem der vorherigen Ansprüche, wobei die erste Flüssigkeit ein pflanzliches oder tierisches Öl oder Fett ist.

19. Verfahren nach einem der vorherigen Ansprüche, wobei die erste Flüssigkeit bei 20°C ein Feststoff, insbesondere ein Wachs, ist.

20. Verfahren nach einem der Ansprüche 1 bis 17, wobei die erste Flüssigkeit ein Treibstoff für einen Verbrennungsmotor ist.

21. Verfahren nach einem der vorherigen Ansprüche, wobei die erste Flüssigkeit ein mineralisches Fett, ein Mineralöl oder ein Mineralöl-Destillat oder -Destillatrückstand, insbesondere Dieselkraftstoff, Leichtöl, Schweröl, Toluol, Benzol oder Benzin, ist.

## Claims

1. A method for dissolving charged nucleic acids in a water-immiscible organic first liquid comprising the following steps:
a) provision of a solution of the nucleic acids in an aqueous second liquid,
b) precipitation of the nucleic acids by adding to the second liquid a complexing agent which forms insoluble complexes with the nucleic acids in the second liquid,
c) removal of the complexes from the second liquid,
d) dissolution of the complexes in a third liquid which consists of an amphiphilic compound or comprises an amphiphilic compound, and
e) mixing of the third liquid comprising the nucleic acids with the first liquid.

2. The method as claimed in claim 1, wherein the complexing agent is a cationic detergent or an organic amine, in particular a quaternary amine.

3. The method as claimed in claim 2, wherein the quaternary amine is cetyltrimethylammonium bromide (CTAB).

4. The method as claimed in any of claims 2-3, wherein the complexing agent is added to the second liquid in step b) in dissolved form.

5. The method as claimed in any of the preceding claims, wherein the nucleic acids are synthetically prepared nucleic acids having a known sequence.

6. The method as claimed in any of the preceding claims, wherein the nucleic acids each have a chain length of from 5 to 100 nucleotides, preferably 10 to 80 nucleotides, particularly preferably 15 to 60 nucleotides.

7. The method as claimed in any of the preceding claims, wherein the nucleic acids consist of DNA or of RNA.

8. The method according to that of the preceding claims, wherein the nucleic acids are antisense DNAs or siRNAs.

9. The method according to that of the preceding claims, wherein the nucleic acids comprise at least a part of a sequence of a human gene.

10. The method as claimed in any of the preceding claims, wherein the nucleic acids have a single-stranded or doublestranded configuration.

11. The method as claimed in any of the preceding claims, wherein the removal of the complexes in step c) takes place by centrifugation or filtration.

12. The method as claimed in any of the preceding claims, wherein the amphiphilic compound is an organic solvent.

13. The method as claimed in any of the preceding claims, wherein the amphiphilic compound comprises at least one ether group.

14. The method as claimed in any of the preceding claims, wherein the amphiphilic compound comprises at least one hydroxyl group.

15. The method as claimed in any of the preceding claims, wherein the amphiphilic compound can be described by the formula HO-R1-0-R2, wherein R1 and R2 is in each case a hydrocarbon residue having 1 to 100 carbon atoms.

16. The method as claimed in any of the preceding claims, wherein the amphiphilic compound is ethylene glycol monobutyl ether, ethylene glycol monoethyl ether or ethylene glycol monomethyl ether.

17. The method as claimed in any of the preceding claims, wherein the complexes are dissolved in the third liquid in an amount such that a nucleic acid concentration in the third liquid which is greater than 0.1 mg/ml, in particular greater than 1 mg/ml, preferably greater than 10 mg/ml, results therefrom.

18. The method as claimed in any of the preceding claims, wherein the first liquid is a vegetable or animal oil or fat.

19. The method as claimed in any of the preceding claims, wherein the first liquid is a solid at 20°C, in particular a wax.

20. The method as claimed in any of claims 1 to 17, wherein the first liquid is a fuel for an internal combustion engine.

21. The method as claimed in any of the preceding claims, wherein the first liquid is a mineral fat, a mineral oil or a mineral oil distillate or distillate residue, in particular diesel fuel, light oil, heavy oil, toluene, benzene or gasoline.

## Revendications

1. Procédé de dissolution d'acides nucléiques chargés dans un premier fluide organique non miscible à l'eau, comprenant les étapes suivantes:
a) mise à disposition d'une solution des acides nucléiques dans un deuxième fluide aqueux,
b) précipitation des acides nucléiques par ajout d'un formateur de complexes formant des complexes insolubles dans le deuxième fluide avec les acides nucléiques au deuxième fluide,
c) séparation des complexes du deuxième fluide,
d) dissolution des complexes dans un troisième fluide qui se compose d'un composé amphiphile ou contient un composé amphiphile, et
e) mélange du troisième fluide contenant les acides nucléiques au premier fluide.

2. Procédé selon la revendication 1, dans lequel le formateur de complexes est un détergent cationique ou une amine organique, notamment une amine quaternaire.

3. Procédé selon la revendication 2, dans lequel l'amine quaternaire est le bromure de cétyltriméthylammonium (CTAB).

4. Procédé selon une des revendications 2 et 3, dans lequel le formateur de complexes est ajouté au deuxième fluide à l'étape b) sous forme dissoute.

5. Procédé selon une des revendications précédentes, dans lequel les acides nucléiques sont des acides nucléiques fabriqués par voie synthétique avec une séquence connue.

6. Procédé selon une des revendications précédentes, dans lequel les acides nucléiques présentent chacun une longueur de chaîne de 5 à 100 nucléotides, de préférence 10 à 80 nucléotides, de manière particulièrement préférée 15 à 60 nucléotides.

7. Procédé selon une des revendications précédentes, dans lequel les acides nucléiques se composent d'ADN ou d'ARN.

8. Procédé selon une des revendications précédentes, dans lequel les acides nucléiques sont des ADN antisens ou ARNsi.

9. Procédé selon une des revendications précédentes, dans lequel les acides nucléiques contiennent au moins une partie d'une séquence d'un gène humain.

10. Procédé selon une des revendications précédentes, dans lequel les acides nucléiques sont réalisés à simple brin ou à double brin.

11. Procédé selon une des revendications précédentes, dans lequel la séparation des complexes selon l'étape c) s'effectue au moyen d'une centrifugation ou filtration.

12. Procédé selon une des revendications précédentes, dans lequel le composé amphiphile est un solvant organique.

13. Procédé selon une des revendications précédentes, dans lequel le composé amphiphile contient au moins un groupe éther.

14. Procédé selon une des revendications précédentes, dans lequel le composé amphiphile contient au moins un groupe hydroxyle.

15. Procédé selon une des revendications précédentes, dans lequel le composé amphiphile peut être décrit par la formule HO-R1-0-R2, dans laquelle R1 et R2 sont chacun un résidu hydrocarbure avec 1 à 100 atomes de carbone.

16. Procédé selon une des revendications précédentes, dans lequel le composé amphiphile est l'éther monobutylique d'éthylèneglycol, l'éther monoéthylique d'éthylèneglycol ou l'éther monométhylique d'éthylèneglycol.

17. Procédé selon une des revendications précédentes, dans lequel les complexes dans le troisième fluide sont dissous dans une quantité telle qu'une concentration en acide nucléique qui est supérieure à 0,1 mg/ml, notamment supérieure à 1 mg/ml, de préférence supérieure à 10 mg/ml, en résulte dans le troisième fluide.

18. Procédé selon une des revendications précédentes, dans lequel le premier fluide est une huile ou graisse végétale ou animale.

19. Procédé selon une des revendications précédentes, dans lequel le premier fluide est un solide à 20°C, notamment une cire.

20. Procédé selon une des revendications 1 à 17, dans lequel le premier fluide est un carburant pour un moteur à combustion.

21. Procédé selon une des revendications précédentes, dans lequel le premier fluide est une graisse minérale, une huile minérale ou un distillat ou résidu de distillat d'huile minérale, notamment du carburant diesel, de l'huile légère, de l'huile lourde, du toluène, du benzène ou de l'essence.


### SEQUENZPROTOKOLL

<110> identif GmbH
<120> Verfahren zum Lösen geladener Nukleinsäuren in einer organischen Flüssigkeit
<130> P75162
<160> 3
<170> PatentIn version 3.4
<210> 1
   <211> 62
   <212> DNA
   <213> Artificial
<220>
   <223> synthetische DNA
<400> 1
   gatcctgcat aatggtagta gtcggccggt acgtatcacc tatacgatgt cagcagatta 60
   tg 62
<210> 2
   <211> 22
   <212> DNA
   <213> Artificial
<220>
   <223> Primer
<400> 2
   cataatctgc tgacatcgta ta 22
<210> 3
   <211> 21
   <212> DNA
   <213> Artificial
<220>
   <223> Primer
<400> 3
   gatcctgcat aatggtagta g 21